Europäisches Patentamt

European Patent Office

Office européen des brevets

(19)

(11) Publication number: **0 244 933**
**B1**

# EUROPEAN PATENT SPECIFICATION

(45) Date of publication of the patent specification:
22.08.90

(21) Application number: 87302145.5

(22) Date of filing: 12.03.87

(51) Int. Cl.⁵: **A61B 5/103**, A61B 5/22

(54) Grip rate measurement.

(30) Priority: 12.03.86 AU 4980/86

(43) Date of publication of application:
11.11.87 Bulletin 87/46

(45) Publication of the grant of the patent:
22.08.90 Bulletin 90/34

(84) Designated Contracting States:
AT BE CH DE ES FR GB GR IT LI LU NL SE

(56) References cited:
GB-A- 1 587 191
GB-A- 2 027 901

MEDICAL & BIOLOGICAL ENGINEERING &
COMPUTING, vol. 20, no. 6, November 1982,
pages 772-774, Stevenage, Herts, GB; "Technical note
Meek grip analyser"

(73) Proprietor: BENJAMIN DEXTRONICS PTY. LIMITED,
89 Bronte Road, Bondi Junction New South Wales(AU)

(72) Inventor: Haski, Andre Leon, 224 Military Road, Dover
Heights New South Wales(AU)

(74) Representative: Simpson, Ronald Duncan Innes et al,
A.A.Thornton & Co. Northumberland
House 303-306 High Holborn, London WCIV 7LE(GB)

## Description

### Field of the Invention

The present invention relates to the detection and measurement of disorders of joint function by the measurement of the rate of movement of a joint against a resistance. Whilst the preferred embodiment of the invention to be described below particularly applies to the assessment of hand function and grip, the present invention is also applicable to different body joints.

### Background of the Invention

It is known that patients suffering from Repetitive Strain Injury, Rheumatoid Arthritis, Multiple Sclerosis, Strokes, head injuries with hemipareses and neuro-muscular diseases, and like diseases suffer from an alteration in the ability to physically grip an object.

The limitations of human static hand function measurement, lie in their inability to measure the hand as an integrated dynamic total system. Various instruments had been evolved to measure particular aspects of hand function such as grip-strength to the ninety-fifth percentile or stiffness within a joint such instruments permitted the demonstration of loss of grip-strength or stiffness within a joint, but did not go far enough towards demonstrating why there had been a loss of function.

The weakness of earlier instruments lay also in their inability to provide a clinical measure of hand function in the total sense. While they could be said to measure particular aspects of hand function and were therefore of use in monitoring rheumatic diseases, they were not capable of picking up the interrelatedness of muscle, tendon and joint function and dysfunction. Such limitations reduced their usefulness as a monitor of integrated hand function.

It has long been known to measure the pressure in a compressible object as a function of time by means of chart recorders and like instruments. It is also known from Australian Patent No. 516026 (to which U.S. Patent No. 4231255 Haski et al corresponds) to provide an instrument which measures the elapsed time between the achievement of predetermined pressure values during the compression of a bladder. The apparatus disclosed in the above mentioned patent provided an instrument which enabled a medical practitioner to determine, by skilled interpretation of the numerical pressure/time data provided, the progress of treatment for a muscular disorder by ascertaining at an early date whether the patient was responding to the treatment.

Notwithstanding the improvement in clinical assessment provided by the above instrument, there exists a need to measure and assess the actual loss of function in a joint or joints in an objective and quantitative manner. For example, in some forms of medical conditions such as Repetitive Strain Injury, the symptoms experienced by the patient may in some instances be perceived mentally rather than actually experienced and the ability to objectively and quantitatively measure any loss of function would therefore greatly enhance the assessment of such persons. Similarly, a method of objectively and quantitatively measuring joint function would also assist in accident cases where one of two hands, for example, has been damaged and financial damages are to be assessed.

### Brief Summary of the Invention

As a result of extensive research flowing from the inventor's previous invention described in the above numbered patents, it has been determined that by measuring a new parameter, which the inventor has called "grip rate", or the rate at which a hand, or other joint, grips a compressible object, the functionality of the hand or joint under consideration can be assessed in an essentially objective and quantitative manner. This new parameter will enable a medical practitioner to determine whether the systems experienced by a patient are muscular, neurological, joint stiffness related or psychosomatically induced, and the appropriate treatment can then be devised.

It is therefore an object of the present invention to provide an apparatus and method for objectively and quantitatively measuring the movement of a joint against a resistance, and measuring the grip rate, or the rate at which a patient grips, in order to provide an improved facility for assessment of treatment.

According to one aspect of the present invention, there is provided an apparatus for measuring the rate at which movement of a joint compresses a compressible object comprising a fluid filled compressible object, such as an inflatable bladder, a pressure transducer in fluid communication with said object having output means connected to circuitry capable of timing specified changes in pressure and calculations including calculating rates of change with time and display means connected to said circuitry to display results of said timings and calculations, characterized in that said circuitry comprises means for determining the elapsed time for said pressure to reach a predetermined lower pressure level, a predetermined upper pressure level and a third predetermined intermediate pressure level, means for calculation of the rate of change with time of the logarithm of the pressure of the fluid in said object between said predetermined lower and upper pressure levels between which said rate of change is substantially linear, and means for calculating the correlation coefficient of the linear portion of said rate of change whereby the effectiveness of the compression of the compressible object may be assessed, the circuit means also records the elapsed time at which the predetermined lower and upper pressures, and a third predetermined intermediate pressure, are attained during a substantially linear portion of the log pressure v time function, and calculates the correlation coefficient of this linear portion to thereby give a measure of the "straightness" or "linearity" of the linear portion which provides the operator with a means of assesing the efficiency of the grip-action of the patient. In a preferred form of the invention, the circuit means may be arranged to allow the operator to select any one of a number of predetermined pressures, and in the case of the assessment of a hand function, the pressures for a relatively normal hand may be 8, 16 and 24 kPa (60, 120 and 180 mm Hg) whereas for a hand known to be affected say by pneumatoid arthritis, the pressure levels 6, 8 and 16 kPs (45, 60 and 120 mm Hg) would be selected.

According to another aspect of the present invention, there is provided a method of assessing the functionality of a joint or joints, comprising compressing a fluid containing bladder by movement of the joint(s), monitoring and timing specified changes in pressure, performing calculations including calculating rates of change with time and displaying results of said timings and calculations, characterized in that said timing includes determining the elapsed time for said pressure to reach a predetermined lower pressure level, a predetermined upper pressure level and a third predetermined intermediate pressure level, that said calculations include calculating the logarithm of said pressure, calculating the rate of charge with time of said logarithm as the pressure changes between said predetermined lower and upper pressure levels between which said rate of charge is substantially linear, and calculating the correlation coefficient of the linear portion of said rate of charge whereby the effectiveness of the compression of the compressible object may be assessed.

## Brief Description of the Drawings

One embodiment of the present invention will now be described with reference to the accompanying drawings in which:

Figure 1 is a schematic representation of an apparatus embodying the invention, and
Figure 2 is a schematic representation showing the relationship between the pressure produced by the apparatus of Figure 1 as a function of time.

## Description of the Preferred Embodiments

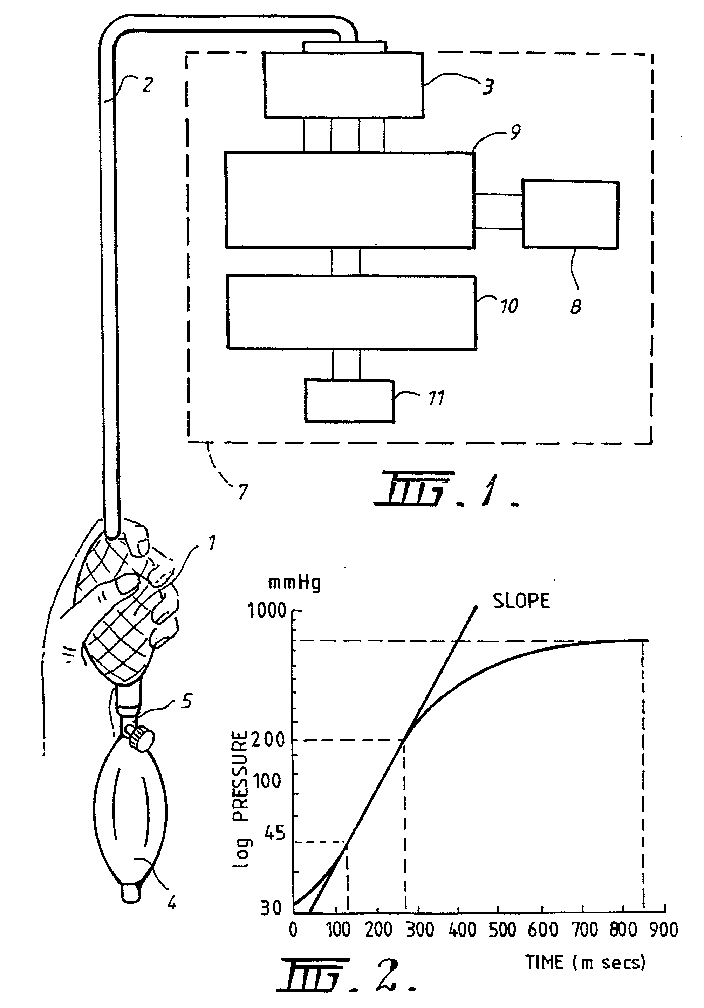

The apparatus shown in Figure 1 of the drawings is one preferred embodiment for the assessment of hand function although it will be appreciated that the principles of that apparatus are equally applicable to the assessment of the functionality of other joints. The apparatus shown consists of an inflatable bladder 1 connected by means of a flexible tube 2 to a pressure transducer 3, such as a Type LX 1602G, manufactured by National Semiconductors.

The bladder 1 is inflatable by means of a squeeze pump 4 and valve 5, such as are normally used on a sphygmomanometer used by doctors in the measurement of blood pressure. In the present embodiment, the bladder 1 is generally pear-shaped and comprises a sygmoidoscope double-bellows in which the one-way valve is replaced by a pressure release valve. A bladder of this shape has been found to provide more consistent readings than other bladder shapes and allowed the small muscles of the hand and the long muscles of grip, as well as the full function of all joints involved to be assessed. When inflated to about 25mm Hg, the bladder is about 10cm long, 7 cm in breath and 4 cm in depth is palm-sized and is almost spherical in cross-section. The bladder 1 is then preferably retained within a mesh or net 6 so that when inflated it retains the shape imposed upon it by the net 6 and does not balloon outwardly at its ends when compressed. The bladder 1 is gripped by the patient in the manner shown in Figure 1.

The pressure transducer 3 is mounted in a casing 7 which also contains the circuitry for analysing the changes in pressure in the fluid in the bladder 1 as it is gripped by the patient. That circuitry is shown schematically in Figure 1 of the drawings and will be seen to comprise a power supply 8, comprising a rechargeable battery and a mains operated supply of standard configuration, a programmable integrated circuit, such as a CMOS programmable array, connected to the output from the pressure transducer 3, and which includes a timer, a central controller 10, such as Type 6502 integrated circuit, which drives three liquid crystal display (LCD) 11 capable of alternately displaying two screens by actuation of a toggle switch (not shown). If necessary, a reset button (not shown) is provided to reset the circuitry before each reading is taken. Similarly, a toggle switch (not shown), is provided to enable the operator to select different pressure ranges as will be explained below.

The programmable integrated circuit 9 may be replaced by a special purpose integrated circuit, and in either case the circuit is programmed or structured to analyze the pressure and time signals to provide (1) a maximum pressure output, (2) outputs of the elapsed time for the pressure to reach predetermined levels, (3) calculation of the logarithm of the pressure signal by any suitable mathemathical method, (4) calculation of the time rate of change of the logarithm of the pressure signal between predetermined lower and upper pressure levels, and (5) calculation of the correlation coefficient of the linear portion of the log pressure v time function between said predetermined pressure levels. In the present embodiment, the programmable integrated circuit 9 is programmed to record the elapsed times for the pressure signal to reach 60, 120 and 180 mm Hg or to reach 45, 60, 120 mm Hg. The alternative elapsed times are outputed by the circuit 9 in accordance with the position of the toggle switch referred to above which is selected by the operator according to the patient being assessed. Where the patient is known to have an affected hand, such as by Rheumatoid Arthritis, the lower pressure levels are selected since the higher levels will not be achievable by such patients.

In operation, the bladder 1 is inflated by the squeeze pump 4 and valve 5 to an initial pressure which is typically about 25 mm Hg. The bladder is then grasped by the patient and squeezed as firmly and as rapidly as possible resulting in an exponential change in pressure of the fluid within the bladder 1. Figure 2 illustrates schematically a typical result for a normal person, the pressure being represented on the ordinate logarithmically. A logarithmic scale was chosen for the pressure ordinate since research data showed that the change in pressure was substantially logarithmic in nature.

The schematic curve illustrated in Figure 2 of the drawings will be seen to include a small initial non-linear portion due to the inertia of the bladder and like effects, a substantially linear region between about 45 and 200 mm Hg, and a plateau or maximum pressure which is reached after about 500 to 600 milliseconds.

The display 11 indicates on its first screen the maximum grip strength in mm Hg or maximum pressure achieved, which is typically in the vicinity of 800 mm Hg, as well as the elapsed time for the pressure to reach the three predetermined pressures referred to above. When the toggle switch is actuated, the second screen of display 11 indicates the magnitude of the grip rate, that is, the slope of the straight line shown in Figure 2 of the drawings, in log mm Hg/mS, the correlation coefficient of the straight line and the maximum grip strength is again displayed. The displays are preferably alpha-numeric, and the values displayed may be identified in an abbreviated manner such as grip rate = Gr Rate, Correlation Coefficient = C Coeff and maximum grip strength = MGS

As mentioned above, the correlation coefficient is used to inform the operator whether the bladder 1 has been gripped properly by the patient. Lack of correlation or lack of linearity in the linear portion of the log pressure v time function indicates an ineffective grip-action on the part of the patient. This is irrespective of the nature of the patient, it being understood that the lower pressure levels for the linear portion are selected in the case of a diseased hand. Where the correlation coefficient is of the order of 0.7 or 0.8, the operator will be aware that the grip action has not been effective.

It will be apparent from Figure 2 that the pressure curve for normal subjects is substantially linear between approximately 45 and 200 mm Hg pressure and thus the slope of the straight line between these pressures conveniently represents grip rate, which is the time rate of change of the logarithmic pressure. However, as mentioned above these upper and lower pressure limits are different for diseased subjects.

It has been experimentally determined that the grip rate of individual patients is remarkably independent of age, strength and sex being approximately 0.04 log mm Hg/mS and in normal cases the grip rate achievable by the left hand is substantially identical to that achievable by the right hand.

This is a very important experimental finding since it enables the damage done to, say, a damaged left hand, to be assessed by measuring the grip rate of the damaged left hand and comparing it with the grip rate of the individual's undamaged right hand which is thus presumed to be the grip rate of the originally undamaged left hand. In this way, the percentage of the damage done to a partially damaged left hand can be assessed. This is of particular importance where the damage is permanent and financial assessment of the damage is required for legal or insurance purposes. It is thought that an analogous result will apply to other body joints such as the knee and elbow. However, for use in relation to these joints the apparatus requires modification by the use of springs and strain gauges rather than the above described bladder and pressure transducers.

A number of physiological experiments have been conducted to determine the sensitivety of the apparatus of the invention in measuring controlled altered states interfering with grip. The behaviour of grip rate and grip strength as measured by the apparatus under conditions of (a) venous congestion, (b) ischaemia, and (c) splinting has been investigated. In each of these experiments, the right hand was used as a control while the left hand was restricted or modified. In each case, a significant deterioration in both the grip rate and the grip strength of the left hand as measured by the apparatus of the present embodiment was detected. The experiments therefore show that the apparatus may be used to objectively and quantitatively assess the function of the hand and other joints.

It will be apparent to those skilled in the art that the apparatus of the present invention will be used by Rheumatologists, Neurologists, Occupational Therapists, Physiotherapists, Medico-Legal Practitioners, and other Medical Officers.

The apparatus has obvious application in the assessment monitoring of diseases such as the treatment of hand injuries - be it injuries to nerve, tendon or muscle involved in hand function. Also it can be used in monitoring the extent of function loss incurred from these injuries or from Poliomyelitis, rheumatoid arthritis or more recently Repetitive Strain Injuries. neck Injuries involving the brachial plexus can be assessed, as well as chronic diseases such as rheumatoid arthritis, Parkinson's disease or even syringomyelia. Myxoedema effect and its response to therapy can also be monitored with this instrument.

The apparatus has obvious application in the monitoring of treatment of hand injuries, tendon, nerve and muscle injuries whether of traumatic origin, or viral origin as in the case of polio. Neck injuries such as brachial plexus damage are also able to be monitored with the apparatus. Other applicable diseases include rheumatoid arthritis, carpal tunnel disease, cerebral ischaemic diseases including Parkinson's disease, metabolic diseases as in myxoedema or myasthenia gravis, and neoplastic diseases as in brain tumours, or congenital diseases of the central nervous system such as Syringomyelia.

The foregoing describes only one embodiment of the present invention and modifications, obvious to those skilled in the art, can be made thereto without departing from the scope of the present invention. For example, the pressure transducer 3 can be replaced by a strain gauge system where it is intended to measure the grip rate of different joints. Similarly, the display 11 may be replaced by any desired number of digital or analogue meters or by a chart recording device. Preferably, provision is made from a chart recorder to be attached to the apparatus described above so that a permanent record of the results of a test may be kept. Other muscle resistance devices can also be substituted for the bladder 1.

## Claims

1. An apparatus for measuring the rate at which movement of a joint compresses a compressible object (1) comprising a fluid filled compressible object such as an inflatable bladder, a pressure transducer (3) in fluid communication with said object having output means connected to circuitry (9, 10) capable of timing specified changes in pressure and calculations including calculating rates of change with time and display means (11) connected to said circuitry to display results of said timings and calculations, characterized in that said circuitry (9, 10) means for determining the elapsed time for said pressure to reach a predetermined lower pressure level, a predetermined upper pressure level and a third predetermined intermediate pressure level, means for calculation of the rate of change with time of the logarithm of the pressure of the fluid in said object between said predetermined lower and upper pressure levels between which said rate of change is substantially linear, and means for calculating the correlation coefficient of the linear portion of said rate of change whereby the effectiveness of the compression of the compressible object may be assessed.

2. The apparatus of claim 1, wherein said predetermined lower, intermediate and upper pressure levels are 8, 16 and 24 kPa (60, 120 and 180 mm Hg) respectively for the testing of relatively normal hand functions whereas said predetermined pressure levels are 6, 8 and 16 kPa (45, 60 and 120 mm Hg) respectively for affected hand functions.

3. The apparatus of claim 1 or 2, further comprising circuit means to display or record the maximum pressure reached during each test.

4. A method of assessing the functionality of a joint or joints, comprising compressing a fluid containing bladder by movement of the joint(s), monitoring and timing specified changes in pressure, performing calculations including calculating rates of change with time and displaying results of said timings and calculations, characterized in that said timing includes determining the elapsed time for said pressure to reach a predetermined lower pressure level, a predetermined upper pressure level and a third predetermined intermediate pressure level, that said calculations include calculating the logarithm of said pressure, calculating the rate of change with time of said logarithm as the pressure changes between said predetermined lower and upper pressure levels between which said rate of change is substantially linear, and calculating the correlation coefficient of the linear portion of said rate of change whereby the effectiveness of the compression of the compressible object may be assessed.

## Patentansprüche

1. Gerät zum Messen der Geschwindigkeit, mit der die Bewegung eines Gelenks einen kompressiblen Gegenstand (1) zusammendrückt, enthaltend ein mit einem Fluid gefüllten, kompressiblen Gegenstand, beispielsweise einen aufblasbaren Balg, einen Druckwandler (3), der mit dem genannten Gegenstand in Fluidverbindung ist und der Ausgabeeinrichtungen aufweist, die mit einer Schaltung (9, 10) verbunden sind, die in der Lage ist, den Zeitverlauf vorbestimmter Druckänderungen zu erfassen und Berechnungen, einschließlich Änderungsgeschwindigkeiten über der Zeit, auszuführen, und eine Anzeigeeinrichtung (11), die mit der Schaltung verbunden ist, um Ergebnisse des Zeitverlaufs und der Berechnungen anzuzeigen, dadurch gekennzeichnet, daß die Schaltung (9, 10) eine Einrichtung enthält, um die Zeit zu ermitteln, die verstreicht, bis der Druck einen vorbestimmten unterern Druckpegel, einen vorbestimmten oberen Druckpegel und einen dritten, vorbestimmten Druckzwischenpegel erreicht, ferner eine Einrichtung zum Berechnen der Änderungsgeschwindigkeit über der Zeit eines Logarithmus des Drucks des Fluides in dem Gegenstand zwischen den vorbestimmten unteren und oberen Druckpegeln aufweist, zwischen denen die Änderungsgeschwindigkeit im wesentlichen linear ist, und eine Einrichtung aufweist zum Berechnen des Korrelationskoeffizienten des lineraren Abschnitts der genannten Änderungsgeschwindigkeit, wodurch die Wirksamkeit der Kompression des kompressiblen Gegenstandes abgeschätzt werden kann.

2. Gerät nach Anspruch 1, bei dem die vorbestimmten unteren, Zwischen- und oberen Druckpegel 8, 16 bzw. 24 kPa (16, 120 und 180 mm Hg) für das Testen relativ normaler Handfunktionen sind, während die genannten vorbestimmten Druckpegel 6, 8 bzw. 16 kPa (45, 60 und 120 mm Hg) für beeinträchtigte Handfuktionen sind.

3. Gerät nach Anspruch 1 oder 2, weiterhin enthaltend eine Schaltungseinrichtung zur Anzeige oder Aufzeichnung des während jeden Tests erreichten Maximaldrucks.

4. Verfahren zur Abschätzung der Funktionsfähigkeit eines Gelenks oder von Gelenken, umfassend das Komprimieren eines ein Fluid enthaltenden Balgs durch Bewegung des Gelenks, bzw. der Gelenke, Beobachten und Erfassen des Zeitverlaufs vorgegebener Druckänderungen, Ausführen von Berechnungen, einschließlich von Änderungsgeschwindigkeiten über der Zeit, und Anzeigen der Ergebnisse der Zeitverläufe und der Berechnungen, dadurch gekennzeichnet, daß das Erfassen des Zeitverlaufs das Ermitteln der Zeit umfaßt, die verstreicht, bis der Druck einen vorbestimmten unteren Druckpegel, einen vorbestimmten oberen Druckpegel und einen dritten vorbestimmten Druckzwischenpegel erreicht, daß die Berechnungen das Berechnen des Logarithmus des Drucks, das Berechnen der Änderungsgeschwindigkeit über der Zeit des Logarithmus, wenn der Druck sich zwischen den vorbestimmten unteren und oberen Druckpegeln ändert, zwischen denen die

**Revendications**

1. Appareil de mesure de la vitesse avec laquelle le mouvement d'une articulation comprime un objet compressible (1), comprenant un objet compressible rempli d'un fluide, par exemple une vessie gonflable, un transducteur de pression (3) qui communique avec ledit objet et qui comporte un dispositif de sortie connecté à un circuit (9, 10) capable de déterminer les moments de changements spécifiés de pression et d'effectuer des calculs, notamment le calcul des vitesses de variation au cours du temps, et un dispositif d'affichage (11) connecté au circuit et destiné à afficher les résultats des calculs et les temps, caractérisé en ce que le circuit (9, 10) comporte un dispositif destiné à déterminer le temps qui s'écoule avant que la pression n'atteigne un niveau inférieur prédéterminé de pression, un niveau supérieur prédéterminé de pression et un troisième niveau intermédiaire prédéterminé de pression, un dispositif de calcul de la vitesse de variation du logarithme de la pression du fluide au cours du temps dans ledit objet, entre les niveaux inférieur et supérieur prédéterminés de pression entre lesquels la vitesse de variation est pratiquement linéaire, et un dispositif de calcul du coefficient de corrélation de la partie linéaire de la vitesse de variation afin que l'efficacité de la compression de l'objet compressible puisse être évaluée.

2. Appareil selon la revendication 1, dans lequel les niveaux inférieur, intermédiaire et supérieur prédéterminés de pression sont égaux à 8, 16 et 24 kPa (60, 120 et 180 mm de mercure) respectivement dans le cas de l'évaluation du fonctionnement d'une main relativement normale, alors que les niveaux prédéterminés de pression sont de 6, 8 et 16 kPa (45, 60 et 120 mm de mercure) respectivement pour le fonctionnement d'une main affectée.

3. Appareil selon la revendication 1 ou 2, comprenant en outre un circuit destiné à afficher ou enregistrer la pression maximale atteinte au cours de chaque essai.

4. Procédé d'évaluation des possibilités de fonctionnement d'une ou plusieurs articulations, comprenant la compression d'une vessie contenant un fluide par un mouvement de l'articulation ou des articulations, le contrôle et la détermination des temps de changements spécifiés de pression, l'exécution de calculs comprenant le calcul de vitesse de variation au cours du temps, et l'affichage des résultats des temps et des calculs, caractérisé en ce que la détermination des temps comprend la détermination des temps écoulés avant que la pression n'ait atteint un niveau inférieur prédéterminé, un niveau supérieur prédéterminé et un troisième niveau intermédiaire prédéterminé de pression, et en ce que les calculs comprennent le calcul du logarithme de la pression, le calcul de la vitesse de variation du logarithme au cours du temps lorsque la pression varie entre les niveaux inférieur et supérieur prédéterminés de pression entre lesquels la vitesse de variation est pratiquement linéaire, et le calcul du coefficient de corrélation de la partie linéaire de la vitesse de variation afin que l'efficacité de la compression de l'objet compressible puisse être évaluée.

EP 0 244 933 B1

$$\mathbb{H}\mathbb{G} . 1 .$$

$$\mathbb{H}\mathbb{G} . 2 .$$